Europäisches Patentamt

European Patent Office

Office européen des brevets

(19)

(11) Veröffentlichungsnummer: **0 501 356 A1**

# EUROPÄISCHE PATENTANMELDUNG

(12)

(21) Anmeldenummer: **92103022.7**

(51) Int. Cl.5: **C12Q 1/68**

(22) Anmeldetag: **22.02.92**

(30) Priorität: **28.02.91 DE 4106251**

(43) Veröffentlichungstag der Anmeldung:
**02.09.92 Patentblatt 92/36**

(84) Benannte Vertragsstaaten:
**AT BE CH DE DK ES FR GB GR IT LI LU NL PT SE**

(71) Anmelder: **BOEHRINGER MANNHEIM GMBH**
**Sandhofer Strasse 116**
**W-6800 Mannheim 31(DE)**

(72) Erfinder: **Kleiber, Jörg, Dr.rer.nat.**
**Unterholzstrasse 22**
**W-8122 Penzberg(DE)**
Erfinder: **Kaletta, Cortina, Dr.rer.nat.**
**Seehauserstrasse 14**
**W-8000 München 70(DE)**
Erfinder: **Kessler, Christoph, Dr.rer.nat.**
**Schlossbergweg 11**
**W-8021 Dorfen(DE)**
Erfinder: **Rüger, Rüdiger, Dr.med.**
**Tutzinger Strasse 2**
**W-8124 Seeshaupt(DE)**

(54) **Nachweis von Bakterien mit Hilfe einer Nukleinsäureamplifikation.**

(57) Verfahren zum spezifischen Nachweis von Bakterien in einer Probe durch Reaktion der Probe mit einem oder mehreren markierten Nukleotidtriphosphaten und einem oder mehreren Enzymen, welche die Herstellung einer markierten Nukleinsäure B katalysieren, die dieses Nukleotid enthält, thermische Denaturierung, Reaktion der Probe mit einer Nukleinsäuresonde C, die hinreichend komplementär zu Nukleinsäure B ist und mindestens eine immobilisierbare Gruppe enthält, Inkontaktbringen des gegebenenfalls gebildeten Nukleinsäurehybrids D mit einer festen Phase, die die immobilisierbare Gruppe erkennt und bindet, Entfernen der flüssigen von der festen Phase und Bestimmung der Markierung an der festen Phase als Maß für die Anwesenheit des Bakteriums.

FIG.1

Gegenstand der Patentanmeldung ist ein Verfahren zum spezifischen Nachweis von Bakterien mittels einer Nukleinsäureamplifikation.

Der Nachweis von Bakterien in der klinischen Diagnostik über Nukleinsäuren und der Molekularbiologie hat in jüngster Zeit gegenüber den klassischen Immuntests immer mehr an Bedeutung gewonnen. Dies hängt damit zusammen, daß Fortschritte im Hinblick auf die Erforschung der Nukleotid-Sequenz von Nukleinsäuren verschiedenen Ursprungs gemacht wurden.

Durch die Einführung von Amplifizierungsverfahren konnte die Empfindlichkeit von Nukleinsäurenachweisen beispielsweise beim Nachweis von Sichelzellenanämie erheblich gesteigert werden. Ein solches Verfahren ist beispielsweise in der EP-A-0 200 362 beschrieben. Der Amplifikationseffekt beruht im wesentlichen darauf, daß aus einer sogenannten target-Nukleinsäure als template mit Hilfe eines Primers und Mononukleotidtriphosphaten ein Verlängerungsprodukt des Primers gebildet wird, das entweder nachgewiesen wird oder selbst wieder als template-Nukleinsäure verwendet werden kann. Dabei kann in das Verlängerungsprodukt auch ein nachweisbares Nukleotid eingebaut werden. Das entstandene Verlängerungsprodukt kann elektrophoretisch nachgewiesen werden.

Diese Nachweismethode ist jedoch wegen des umständlichen und zeitaufwendigen Elektrophoreseschritts nachteilig.

Das nicht markierte Verlängerungsprodukt kann gemäß EP-A-0 201 184 auch durch Hybridisierung mit einer nachweisbar markierten Nukleinsäuresonde nachgewiesen werden. Die Abtrennung des Hybrids aus Verlängerungsprodukt und Sonde von unumgesetzter Sonde ist jedoch mit dem dort beschriebenen Verfahren wegen unspezifischer Wechselwirkungen entweder ineffizient oder mit vielen Waschschritten verbunden, welche die Empfindlichkeit herabsetzen.

In der WO 89/11546 wird ein Verfahren vorgeschlagen, bei dem amplifizierte, festphasengebundene Nukleinsäuren mit einem Primer und markierten Mononukleotiden umgesetzt werden, wobei die dabei gebildeten markierten Nukleinsäuren nachgewiesen werden. Dieses Verfahren hat den Nachteil, daß alle maßgeblichen Reaktionen an der festen Phase ablaufen, wodurch die Reaktionsgeschwindigkeit beeinträchtigt ist. Auch in der EP-A-0 324 474 wird ein solches Verfahren vorgeschlagen, bei dem markierte Mononukleotide eingebaut und diese markierten Nukleinsäuren mit zur nachzuweisenden Nukleinsäure komplementären immobilisierten Nukleinsäuren abgefangen werden. Über die Methode der Denaturierung der amplifizierten Nukleinsäuren und die Hybridisierungsbedingungen ist nichts ausgesagt. Ein weiterer Nachteil dieser Verfahren ist, daß die Herstellung effizient festphasengebundener Nukleinsäuren aufwendig ist.

In der EP-A-0 357 011 wird ein Verfahren beschrieben, bei dem in die Verlängerungsreaktion zwei Primer eingesetzt werden, von denen einer nachweisbar markiert und der andere zur Bindung an eine feste Phase geeignet ist. Dieses Verfahren hat den Nachteil, daß es aufwendiger ist, nachweisbar markierte Oligonukleotide von den diese Oligonukleotide beinhaltenden Verlängerungsprodukten abzutrennen. Wenn keine Abtrennung vorgenommen wird, ist wegen Konkurrenzreaktionen eine verringerte Sensitivität zu erwarten.

In der EP-A-0 297 379 und der EP-A-0 348 529 ist ein Verfahren beschrieben, bei dem ein immobilisierter oder immobilisierbarer Primer unter Zuhilfenahme der target-Nukleinsäure als template mit einem detektierbaren Mononukleotid zu einer immobilisierten und gleichzeitig nachweisbar markierten Nukleinsäure verlängert wird. Dieses Verfahren hat u.a. den Nachteil, daß die Spezifität des Nachweises nicht sehr hoch ist. Das ebenfalls in EP-A-0 297 379 beschriebene Verfahren, bei dem nur ein immobilisierter oder immobilisierbarer Primer eingesetzt wird, woraufhin die Verlängerungsprodukte mit einem markierten Oligonukleotid umgesetzt werden, hat den bei EP-A-0 357 011 beschriebenen Nachteil der erschwerten Abtrennbarkeit des Oligonukleotids.

In der WO 89/09281 ist ein Verfahren beschrieben, bei dem die beiden Primer dieselbe chemische Gruppe aufweisen, die einmal zur Immobilisierung und zum anderen zum Nachweis verwendet wird. Dies verstärkt den oben genannten Nachteil der schlechten Abtrennbarkeit noch.

In Proc. Natl. Acad. Sci. USA, Vol. 86, pp 6230-6234 wird ebenfalls ein Verfahren geschildert, in dem ein nachweisbar markierter Primer verlängert wird. Der Nachweis erfolgt nach Bindung der Verlängerungsprodukte an eine Fangprobe. Auch hier ist die Abtrennung des nachweisbar markierten Primers nicht ohne Zusatzschritte möglich, und er stört daher die Nachweisreaktion.

Ein Nachweisverfahren für Bakterien aus der EP-A-0 131 052 bekannt. In diesem Verfahren werden die bakteriellen Nukleinsäuren mit einer nachweisbar markierten Nukleinsäuresonde, die kürzer ist als die nachzuweisende Nukleinsäure, umgesetzt und anschließend das gebildete Hybrid nachgewiesen. Dieses Verfahren hat den Nachteil, daß es relativ unempfindlich ist und daher eine weitgehende Entfernung von anderen Zellbestandteilen und eine Konzentrierung der nachzuweisenden Nukleinsäuren erfordert.

Es war daher Aufgabe der Erfindung die Nachteile des Verfahrens des Standes der Technik zu vermeiden und insbesondere ein Bakteriennachweisverfahren bereitzustellen, welches hohe Spezifität oder Selektivität mit geringem Hintergrundsignal verbindet.

Gegenstand der Erfindung ist ein Verfahren zum spezifischen Nachweis eines Bakteriums in einer Probe, welches folgende Schritte umfaßt:

a) Aufschluß der Probe zur Freisetzung bakterieller Nukleinsäuren,

b) Reaktion der aufgeschlossenen Probe mit einem oder mehreren markierten Mononukleosidtriphosphaten und einem oder mehreren Enzymen, welche die Herstellung einer markierten Nukleinsäure B katalysieren, die dieses Nukleotid enthält,

c) Reaktion der Probe mit einer für das Bakterium spezifischen Nukleinsäuresonde C, welche hinreichend komplementär zu der Nukleinsäure B ist,

d) Nachweis des aus markierter Nukleinsäure B und Nukleinsäuresonde C gebildeten Nukleinsäurehybrids D,

e) wobei die Nukleinsäuresonde mindestens eine immobilisierbare Gruppe enthält,

f) die Reaktionsmischung direkt vor Schritt c) einer thermischen Denaturierung unterzogen wird,

g) das Nukleinsäurehybrid D mit einer festen Phase, welche die immobilisierbare Nukleinsäuresonde C spezifisch binden kann, in Kontakt gebracht wird,

h) die flüssige von der festen Phase getrennt wird und

i) die an die feste Phase gebundene nachweisbare Gruppe nachgewiesen wird.

Nicht zum Gegenstand der Erfindung gehören sollen Verfahren zum Nachweis von Bakterien gemäß DE-A-39 29 030. Ausgeschlossen sind somit Verfahren, bei denen die bakteriellen Nukleinsäuren mit mindestens zwei Adaptoren pro Nukleinsäurestrang, von denen mindestens einer eine für ein Replikationssystem spezifische Nukleotidsequenz enthält, unter Bildung einer zu der nachzuweisenden Nukleinsäure im wesentlichen komplementären Nukleinsäure, die außerdem mindestens einen Adaptor enthält, umgesetzt werden. Ausgeschlossen sind bevorzugt Nachweisverfahren, die auf einem proteingeprimten Replikationssystem beruhen.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, 2.9.1. - 2.9.10 und Molecular Cloning, Edt. J. Sambrook et al., CSH, 1989, 9.4.7. - 9.5.8. Bezug genommen. Zu den bekannten Methoden gehört auch die Herstellung von markierten Nukleosidtriphosphaten, wie sie in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GC-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe L. Direkt nachweisbare Gruppen sind beispielsweise radioaktive ($^{32}$P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da an mit ihnen markierte Nukleosidtriphosphate im allgemeinen besonders gut als Substrate von Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Nukleosidtriphosphate (NTP) sind Ribo (rNTP)- oder Desoxyribonukleosidtriphosphate (dNTP).

Unter einer target-Nukleinsäure wird eine bakterielle Nukleinsäure verstanden, die Ziel des Nachweises und Ausgangsstoff des erfindungsgemäßen Verfahrens ist.

Eine template-Nukleinsäure A ist eine Nukleinsäure, zu der ein im wesentlichen komplementärer Nukleinsäurestrang neu gebildet wird. In Bezug auf die Sequenzinformation dient die template-Nukleinsäure als Matritze und enthält die Sequenzformation, die in Reaktion b) in die Nukleinsäure B umgeschrieben wird. Die Nukleinsäure A ist entweder die target-Nukleinsäure oder eine davon abgeleitete Nukleinsäure. Sie kann beispielsweise ein Teil der target-Nukleinsäure sein oder einen Teil der target-Nukleinsäure neben anderen Teilen enthalten, beispielsweise hochkomplexe Nukleinsäuren. Sie kann auch einen Teil des zu der target-Nukleinsäure komplementären Stranges enthalten.

Denaturierung bedeutet Auftrennung von Nukleinsäuredoppelsträngen in Einzelstränge. Dem Fachmann stehen eine Vielzahl von Varianten zur Verfügung.

Unter einem spezifischen Nachweis wird ein Verfahren verstanden, durch welches gewünschtenfalls selektiv bestimmte Bakterien auch in Gegenwart anderer Bakterien nachgewiesen werden können. Es ist jedoch auch möglich, eine Gruppe von Bakterien mit Nukleinsäuren teilweise übereinstimmender oder ähnlicher Nukleotidsequenz nachzuweisen. Zum Nachweis doppelsträngiger Nukleinsäuren kann jeder der beiden komplementären Stränge einbezogen werden.

Unter einer zu einer Nukleinsäure im wesentlichen komplementären Nukleinsäure oder Nukleinsäuresequenz werden Nukleinsäuren oder Sequenzen verstanden, die mit der entsprechenden Nukleinsäure hybridisieren können, deren Nukleotidsequenz im hybridisierenden Bereich entweder genau komplementär zu der anderen Nukleinsäure ist oder sich in wenigen Basen von der genau komplementären Nukleinsäure unterscheidet. Die Spezifität richtet sich dabei sowohl nach dem Grad der Komplementarität als auch nach den Hybridisierungsbedingungen.

Flüssige Phasen sind die üblicherweise bei Nukleinsäurenachweisen verwendeten wässrigen Phasen mit gelösten organischen bzw. anorganischen Inhaltsstoffen, z.B. Hybridisierungspuffer, überschüssigen Nukleotiden, Nukleinsäuren weiterer nicht nachzuweisender Bakterien, Proteinen etc.

Das erfindungsgemäße Verfahren zum Nachweis von Bakterien beruht auf dem spezifischen Nachweis einer für das nachzuweisende Bakterium spezifischen Nukleinsäure. Bevorzugt liegen diese Nukleinsäuren in Lösung vor. Die Reaktionssequenz wird meist gestartet durch Verfügbarmachung der bakteriellen Nukleinsäure mit entsprechenden Reagentien, dem sogenannten Aufschluß. Hierbei können sowohl physikalische Methoden, wie Einsatz von Scherkräften (z.B. hydrodynamischer Scherkräfte, realisiert durch Frenchpresse, Homogenisator), Ultraschall (Entstehung von Kavitäten), Osmose (hydrostatischer Druck, der gegen die Zellmembran gerichtet ist), Hitze, Wiederholung extremer Temperaturänderungen (Auftauen/Einfrieren) und ionisierende Strahlung, aber auch chemische Methoden, wie Inhibierung der Zellwandsynthese (z.B. durch Antibiotika wiew Penicillin), enzymatischer Zellwandabbau (durch Enzyme, die speziell die Zellwandstruktur angreifen, wie Lysozym, Lysostatin, Proteasen) und Bacteriophagen-Lyse, verwendet werden. Solche Methoden sind beispielsweise beschrieben in Methods in Microbiology, Academic Press, Inc. N.Y., Vol. 5 B, 1971 und Manual of Methods for General Bacteriology, American Society for Microbiology, Chapter 5, 1981.

Da das erfindungsgemäße Verfahren sehr empfindlich und selektiv ist, können auch kleine Nukleinsäuremengen in Anwesenheit anderer Stoffe, beispielsweise Proteinen, Zellen, Zellfragmenten, aber auch nicht nachzuweisender Nukleinsäuren nachgewiesen werden. Eine Aufreinigung von Proben kann somit entfallen, wenn sichergestellt ist, daß die nachzuweisenden Nukleinsäuren für die eingesetzten Reagenzien ausreichend zugänglich sind.

Bei der Untersuchung von Lebensmitteln findet die Freisetzung bevorzugt in einem mehrstufigen Prozess statt. Zunächst wird die zu untersuchende Probe aufgeschlossen, um die nachzuweisenden Bakterien freizusetzen. Bei in sehr kleiner Stückzahl vorkommenden Bakterien werden diese in einem nachfolgenden Schritt in einer Kultur vermehrt. Bei anderen Bakterien ist dies nicht erforderlich. Aus den so erhaltenen Bakterien werden die bakteriellen Nukleinsäuren in bekannter Weise, wie oben beschrieben, durch Lyse freigesetzt. Die Nukleinsäuren können vorbehandelt werden. Zu den bekannten Vorbehandlungen gehört beispielsweise die cDNS-Synthese aus RNS. Damit das erfindungsgemäße Verfahren seine Vorteile voll entfalten kann, hat es sich als zweckmäßig erwiesen, wenn die Nukleinsäure eine Größe von mindestens 40 bp aufweist.

Weiterhin können die Nukleinsäuren das Produkt einer vorgeschalteten spezifischen oder unspezifischen Nukleinsäurevermehrung sein. Solche Nukleinsäureamplifikationsverfahren sind beispielsweise aus
EP-A-0 201 184, EP-A-0 272 098, DE-A-37 26 934, EP-A-0 237 362, WO 88/10315, WO 90/01069, WO 87/06270, EP-A-0 300 796, EP-A-0 310 229, WO 89/09835, EP-A-0 370 694, EP-A-0 356 021, EP-A-0 373 960, EP-A-0 379 369, WO 89/12696 oder EP-A-0 361 983
bekannt.

Die nachzuweisenden (target-)Nukleinsäuren können direkt als template-Nukleinsäuren A in Reaktion b) eingesetzt werden, wenn sie die für das gewählte Enzymsystem erforderlichen Bedingungen erfüllen. Dazu gehört für manche Enzyme, daß es sich um einzelsträngige Nukleinsäuren handelt, bei anderen ist es erforderlich, daß sie Erkennungsstellen bzw. Promotoren für das Enzymsystem beinhalten.

Ist dies nicht der Fall, so müssen die target-Nukleinsäuren in einem Reaktion b) vorgeschalteten Schritt in derartige template-Nukleinsäuren A überführt werden.

Es kann sich bei A um eine Ribonukleinsäure oder eine Desoxyribonukleinsäure handeln. Besonders bevorzugt als Nukleinsäure A sind Desoxyribonukleinsäuren.

Das Enzym E im Sinne der Erfindung ist ein Enzym oder Enzymsystem, das die templatabhängige Synthese von Nukleinsäuren aus Mononukleosidtriphosphaten katalysiert. Bevorzugte Enzyme sind Polymerasen und Transskriptasen, welche die Verknüpfung von Mononukleotiden bewirken. Solche Enzyme sind dem Fachmann bekannt. Ausgeschlossen sind bevorzugt proteingeprimte Replicasen.

In einem Schritt b) wird aus der template-Nukleinsäure A eine markierte Nukleinsäure B hergestellt. Dies kann auf prinzipiell jede Art und Weise geschehen, solange nur die spezifische Information der Nukleotidsequenz der Nukleinsäure A oder eines Teils davon im wesentlichen erhalten bleibt.

Da der erfindungsgemäße Bakteriennachweis bevorzugt Ribonukleinsäuren als target-Nukleinsäuren verwendet, erübrigt es sich im allgemeinen, sie vor oder während der Reaktion b) einzelsträngig zu machen. Eine evtl. gewünschte Strangtrennung kann durch Behandlung mit Alkali, aber auch thermisch, enzymatisch oder mittels chaotroper Salze geschehen.

Die Verwendung von Reaktionen b), die von der Anwesenheit eines spezifischen Starters abhängig sind, ist wegen der erhöhten Spezifität besonders bevorzugt. Solche spezifischen Starter bewirken, daß das Enzym nur auf die Nukleinsäure wirkt, welche diesen Starter gebunden hat. Der Starter ist bevorzugt ein sogenannter spezifischer Primer P1 oder ein Promotor.

Spezifische Primer P1 sind besonders modifizierte oder unmodifizierte Oligonukleotide, die eine im wesentlichen zu der Nukleinsäure A komplementäre Nukleotidsequenz S aufweisen. Modifizierte Oligonukleotide können beispielsweise Gruppen enthalten, die die Hybridisierung des Primers mit der Nukleinsäure nicht wesentlich beeinträchtigen. Solche Oligonukleotide können chemisch oder enzymatisch hergestellt werden. Als template-Nukleinsäure A kann direkt die target-Nukleinsäure eingesetzt werden.

Die Verwendung solcher Primer P1 setzt also voraus, daß zumindest ein Teil der Sequenz der Nukleinsäure bekannt ist. Die Sequenz des Primers wird ferner so gewählt, daß er an einem seiner Enden, bevorzugt am 3'-Ende, kürzer ist als die Nukleinsäure. Diese weist daher über das 3'-Ende des Primers hinaus ein überstehendes einzelsträngiges Teilstück auf.

Anhand der publizierten Nukleotidsequenzen einzelner Bakterien ist danach eine Auswahl von Primersequenzen S möglich (Salmonella: Inf. Immun. 58: 2651 (1990) ; Res. Microbiol. 140: 455 (1989); J. Bacteriol. 173: 86 (1991). Listeria monocytogenes: Mol. Microbiol. 4: 1091 (1990); Infect. Immun. 55: 3225 (1987).

In den Reaktionsschritt b) können pro nachzuweisendem Nukleinsäureeinzelstrang ein oder mehrere spezifische Primer eingesetzt werden. Im Fall mehrerer Primer überlappen sich die Bereiche auf der Nukleinsäure, mit denen die Primer hybridisieren können, bevorzugt nicht, und besonders bevorzugt liegen zwischen diesen Bereichen einzelsträngige Bereiche der Nukleinsäure A. Der Primer kann neben einem zu der template-Nukleinsäure A im wesentlichen komplementären Teil, bevorzugt am 5'-Ende, auch noch eine Nukleotidsequenz S1 beinhalten, die nicht mit der Nukleinsäure insbesondere dem an den komplementären Bereich anschließenden Bereich hybridisieren kann.

Diese Sequenz S1 kann beispielsweise einzel- oder doppelsträngig sein und auch eine Erkennungssequenz für ein Enzym enthalten. Dies kann z.B. eine Restriktionsschnittstelle sein. Auch kann der Primer im Hinblick auf effektives Priming beispielsweise ein Protein gebunden enthalten, welches von einem Enzym E, vorzugsweise einer Polymerase, erkannt wird.

Damit die Reaktion b) ablaufen kann, müssen die Nukleinsäure A und der Primer P1 im komplementären Bereich zunächst jeweils von gegebenenfalls anwesenden Komplementärsträngen getrennt werden. Diese Trennung kann nach bekannten Methoden, beispielsweise thermisch oder nichtthermisch erfolgen. Die nichtthermische Denaturierung ist bevorzugt. Reaktion b) wird dann unter Bedingungen gestartet, bei denen A und P1 miteinander hybridisieren können.

Bei Verwendung eines Primers als Starter wird der Probe außerdem ein Enzym E zugegeben, welches in einer primerabhängigen Reaktion zu A komplementäre Nukleinsäure synthetisieren kann. Dazu gehören insbesondere Polymerasen. Ihre Substratspezifität richtet sich nach der Nukleinsäure A. Ist A RNA, so kommen insbesondere RNA-abhängige DNA-Polymerasen, wie reverse Transkriptase (z.B. aus AMV oder M-MuLV) in Frage. Ist A DNS, so sind DNS-abhängige DNS-Polymerasen, wie Klenow-Enzym oder Taq-DNS-Polymerase bevorzugt.

Ebenfalls der Probe zugegeben werden Desoxyribonukleosidtriphosphate (dNTP), von denen mindestens eines markiert ist.

Produkt der Polymerasereaktion ist eine markierte Desoxyribonukleinsäure B, in welche der Primer P1 sowie das markierte Desoxyribonukleosidtriphosphat eingebaut sind. Diese Nukleinsäure B ist gleich groß oder kleiner als das template, weist jedoch einen Bereich auf, der zu mindestens einem Teil im wesentlichen komplementär zum template ist.

Diese Nukleinsäure B kann nun direkt in Schritt c) eingesetzt werden. Vorteilhaft wird sie jedoch nochmals als template-Nukleinsäure einer Reaktion analog zu Schritt b) unterworfen. Hierzu wird der Probe ein Primer P2 zugesetzt, der im wesentlichen komplementär zu einem Teil der Nukleinsäure B, bevorzugt zu einem Teil des neu aus dNTPs gebildeten Bereiches, ist. Das Primerpaar P1 und P2 erfüllen bevorzugt die in EP-A-0 201 184 geschilderten Bedingungen. Besonders bevorzugt sind sie insbesondere in ihrem zu verlängernden 3'-Ende 100% komplementär zu A bzw. B. Bevorzugt werden P1 und P2 gleichzeitig zu dem template A zugegeben.

Um eine noch höhere Empfindlichkeit zu erlangen, ist es möglich, Reaktion b) noch mehrere Male, bevorzugt 1-60, besonders bevorzugt 20-60 mal, durchzuführen, wobei jeweils die Produkte der Reaktion erneut in Reaktion b) eingesetzt werden. Dadurch ergibt sich theoretisch eine nahezu exponentielle Vermehrung an markierten Nukleinsäuren. Es werden dabei beide Nukleinsäurestränge gebildet, wobei die Länge durch die nicht verlängerbaren Enden der Primer P1 und P2 festgelegt ist. Es ist analog zu EP-A-0 201 184 möglich, in den späteren Durchläufen von Reaktion b) auch sogenannte nested Primer zu verwenden, deren Sequenz so gewählt ist, daß die enstehenden Nukleinsäuren kleiner sind als die zunächst gebildeten. Vor jedem Durchlauf von Reaktion b) ist es zweckmäßig, die in Reaktion b) gebildeten Doppelstränge aus A und B zu trennen. Dies kann thermisch oder nicht thermisch geschehen. Bevorzugt ist die thermische Trennung. Es müssen jeweils erneut Primer P1 und P2 anwesend sein.

Der Starter kann auch ein Promotor sein. Ein Promotor ist eine Nukleotidsequenz, die von einer RNS-Polymerase erkannt wird und diese veranlaßt, einen zu der auf den Promotor folgenden Nukleotidsequenz komplementären Nukleinsäurestrang B zu synthetisieren. Dabei wird neben den nicht markierten NTPs auch das markierte NTP in den gebildeten Nukleinsäurestrang eingebaut.

Geeignete Promotoren sind bekannt, beispielsweise RNS-Polymerase Bindungsstellen aus Bakterienphagen wie T3, T7 oder SP6 (Melton et al.: NAR 12, 1984, 7035-56; Pfeiffer & Gilbert: Protein Sequences and DNA-Analysis I, 1988, 269-280; Uhlenbeck et al.: Nature 328, 1987, 596-600).

Der Promotor ist in einer bevorzugten Ausführungsform des Nachweisverfahrens Teil eines spezifischen Primers zur Herstellung der template-Nukleinsäure A aus der target-Nukleinsäure. Dieser Primer hat eine Nukleotidsequenz S, die im wesentlichen komplementär zu einem Teil der target-Nukleinsäure ist, und eine Nukleotidsequenz S1, die von einer RNS-Polymerase erkannt wird und mindestens eine Promotor-sequenz beinhaltet. In einer ersten Reaktion wird mit dem Primer und einer von der Art der target-Nukleinsäure abhängigen DNS-Polymerase und dNTPs ein zu der target-Nukleinsäure mindestens teilweise komplementärer Nukleinsäurestrang A1 gebildet, der den Primer mit dem Promotor beinhaltet. Falls nicht bereits mit dem Primer verbunden, wird das neu gebildete Nukleinsäurestück durch Zugabe eines weiteren Enzyms, bevorzugt einer Ligase, z.B. E.coli-DNS-Ligase mit dem Primer kovalent verknüpft. Die Ligase kann auch thermisch stabil sein. A1 ist bevorzugt kürzer als die target-Nukleinsäure. Bevorzugt wird in dieser Transkriptionsreaktion ein zweiter zum target-Nukleinsäurestrang komplementärer Primer P3 eingesetzt und die Lücke zwischen beiden Primern geschlossen, beispielsweise durch eine Gap-Filling-Reaktion. Der Einsatz markierter dNTPs ist hier noch nicht erforderlich, da sich diese Maßnahme im erfindungsgemäßen Verfahren in einer nur geringen Verstärkung des Meßsignals auswirkt, aber möglich.

Ist die target-Nukleinsäure RNS, so wird diese bevorzugt in einem folgenden Schritt selektiv abgebaut. Dazu sind bekannte Verfahren geeignet, beispielsweise die Behandlung mit Alkali oder mit RNAsen. Anschließend wird ein zu A1 komplementärer Nukleinsäurestrang A2 gebildet. Bevorzugt wird dazu der Reaktionsmischung ein Primer P2 zugesetzt, der zu einem Teil, bevorzugt einem neu gebildeten Teil des Stranges A1 komplementär ist. Bevorzugt enthält der Primer P2 ebenfalls die Promotorsequenz S2. Dieser wird, wie oben bei A1 beschrieben zu A2 verlängert. Solche Verfahrensschritte sind z.B. in der EP-A-0 329 822, DE-A-37 26 934, WO 88/10315, WO 87/06270, EP-A-0 310 229 und EP-A-0 373 960 beschrieben, weshalb hier auf diese Offenbarungen vollinhaltlich Bezug genommen wird.

Insbesondere in Bezug auf Details, die zur reversen Transkription von RNS oder Transkription von DNS hilfreich und erforderlich sind, wird auf diese Offenbarung verwiesen.

Besonders bevorzugt enthält die Probe bei dieser Ausführungsform außerdem den zu der target-Nukleinsäure komplementären Strang, wobei P1 und P2 gleichzeitig verlängert werden.

Bei der genannten Ausführungsform wird der so vorbehandelten Probe anschließend erfindungsgemäß eine Promotor-spezifisch gesteuerte DNS-abhängige RNS-Polymerase eingesetzt. Solche Polymerasen sind z.B. T3, T7 oder SP6 RNS-Polymerase. Mit ihrer Hilfe werden aus NTPs und dem markierten NTP markierte Nukleinsäuren B gebildet. Die doppelsträngige Nukleinsäure A1/A2 dient dabei als template-Nukleinsäure, bevorzugt mehrmals.

Bevorzugte NTPs sind Ribonukleotidtriphosphate. Da bei dieser Variante der Reaktion b) einzelsträngige Nukleinsäuren B gebildet werden, ist eine Denaturierung zur Strangtrennung nicht unbedingt erforderlich, aber möglich.

Eine bevorzugte Ausführungsform ist das Vorgehen nach DE-A-4010465, jedoch unter Verwendung nachweisbar markierter Ribonukleotidtriphosphate.

Nach dem letzten Durchführen von Schritt b) wird in dem erfindungsgemäßen Verfahren die Reaktionsmischung einer thermischen Denaturierung (Reaktion f)) unterzogen. Auch falls Schritt b) mehrfach durchgeführt wird, findet eine thermische Denaturierung direkt vor Schritt c) statt. Insbesondere werden dabei Nukleinsäuredoppelstränge voneinander getrennt. Thermische Denaturierung bedeutet insbesondere Denaturierung in einem Temperaturbereich von ca. 50-95 °C, bevorzugt 85-95 °C. Die Denaturierung wird bevorzugt 1-15 min. lang durchgeführt.

Die thermische Denaturierung bietet den Vorteil, daß keine zusätzlichen Reagenzien, wie Natronlauge benutzt werden müssen. Damit entfallen Pipettierschritte, das Verfahren wird vereinfacht und die Reproduzierbarkeit des Tests erhöht.

Im folgenden Reaktionsschritt c) wird Nukleinsäure B mit der Sonde C so zur Reaktion gebracht, daß sie zusammen ein Nukleinsäurehybrid D bilden.

Als Nukleinsäuresonde C kommen insbesondere Oligo- bzw. Poly-Nukleotide mit einer Länge von 6 bis 5000, bevorzugt 15 bis 2000 in Frage. Die Sonde C kann ein Plasmid, ein Nukleinsäurefragment oder ein Oligonukleotid sein. Es kann sich um RNS oder DNS handeln. Nukleinsäuresonde C wird im Überschuß zu der erwarteten Menge an Nukleinsäure B zu der Reaktionsmischung vorteilhaft in Form einer wässrigen Lösung zugegeben. Die Sonde C weist eine Nukleotidsequenz auf, die im wesentlichen komplementär zu B ist, und die für B spezifisch ist, also nicht oder nur in sehr geringem Umfang mit in der Probe vorhandenen oder neu gebildeten Nukleinsäuren hybridisiert, die nicht nachgewiesen werden sollen. Durch die Kombination der Verwendung spezifischer Primer und der Hybridisierung mit einer spezifischen Sonde wird das erfindungsgemäße Verfahren besonders selektiv.

C kann eine doppelsträngige Nukleinsäure sein, deren einer Strang C1 komplementär zu einem Teil von B ist. Bevorzugt ist der andere Strang C2 der Nukleinsäuresonde zu anderen Nukleinsäuren B komplementär, insbesondere zu solchen, die bei Durchführung von Reaktion b) mit B als template-Nukleinsäure entstehen. Die Denaturierung von C kann dabei separat von B vorgenommen werden. Es ist jedoch bevorzugt, C zusammen mit B zu denaturieren.

Bevorzugt ist der Fall, daß es sich bei C um eine einzelsträngige Nukleinsäuresonde C1 handelt und der zu C1 komplementäre Strang C2 nicht zugesetzt wird. Es ist dann auch möglich, C1 vor Denaturierung von B zuzugeben. Die Lösung mit der einzelsträngigen Nukleinsäure C1 enthält außerdem bevorzugt zur Hybridisierung hilfreiche Reagenzien, wie z.B. SSC, Formamid oder Blockierungsreagenzien für nicht nachzuweisende Nukleinsäuren. Dadurch entfallen zusätzliche Pipettierschritte für die Zugabe der Hybridisierungslösung.

Einzelsträngige Nukleinsäuresonden C1 können beispielsweise durch chemische Nukleinsäuresynthese gemäß DE-A- 39 16 871 oder auch gemäß EP-B-0 184 056 hergestellt werden.

Sonde C enthält pro Nukleinsäurestrang eine oder mehr zur Immobilisierung befähigende (immobilisierbare) Gruppen I.

Zur Immobilisierung befähigende Gruppen I sind beispielsweise chemische Gruppen, die kovalent, beispielsweise über eine chemische oder eine Photoreaktion an eine feste Phase gebunden werden können, oder Gruppen bzw. Molekülteile, die über gruppenspezifische Wechselwirkungen von einem anderen Molekül oder Molekülteil erkannt und gebunden werden können. Solche Gruppen sind daher z. B. Haptene, Antigene und Antikörper, Nukleotidsequenzen, Rezeptoren, Regulationssequenzen, Glykoproteine, beispielsweise Lectine, oder auch die Bindungspartner von Bindeproteinen, wie Biotin oder Iminobiotin. Bevorzugt sind Vitamine und Haptene, besonders bevorzugt sind Biotin, Fluorescein oder Steroide, wie Digoxigenin oder Digoxin. Für die Erfindung ist es wichtig, daß in jedem Hybrid D die immobilisierbare Gruppe der Sonde sich von der nachweisbaren Gruppe der Nukleinsäure B unterscheidet.

Die Mischung, welche das Nukleinsäurehybrid D enthält, wenn die nachzuweisende Nukleinsäure in der Probe vorhanden war, wird anschließend mit einer festen Phase in Kontakt gebracht, welche das Hybrid D über die immobilisierbaren Gruppen der Nukleinsäuresonde C spezifisch binden kann.

Die Art der Festphase richtet sich nach der zur Immobilisierung befähigenden Gruppe I. Bevorzugt weist sie eine immobilisierende Gruppe R auf, die eine bindende Wechselwirkung mit I eingehen kann. Ist die immobilisierbare Gruppe beispielsweise ein Hapten, dann kann eine Festphase verwendet werden, die an ihrer Oberfläche Antikörper gegen dieses Hapten aufweist. Ist die immobilisierbare Gruppe ein Vitamin, wie z. B. Biotin, dann kann die Festphase bindende Proteine, wie Avidin oder Streptavidin immobilisiert enthalten. Besonders bevorzugte Reste I und R sind Biotin und Streptavidin. Die Immobilisierung über eine Gruppe an der modifizierten Nukleinsäure ist besonders vorteilhaft, da sie unter milderen Bedingungen stattfinden kann als beispielsweise Hybridisierungsreaktionen.

Bevorzugt wird zur Immobilisierung der gebildeten Nukleinsäuren die Reaktionsmischung nach Bildung der Nukleinsäurehybride D in ein Gefäß gefüllt, welches an seiner Oberfläche mit der immobilisierbaren Gruppe reagieren kann. Bevorzugt findet die Hybridisierungsreaktion mit der Sonde gleichzeitig mit der Immobilisierung statt. Das Gefäß kann beispielsweise eine Küvette, ein Röhrchen oder eine Mikrotiterplatte sein. Es ist jedoch auch möglich, eine Festphase in Form eines porösen Materials, wie einer Membran, eines Gewebes oder eines Vlieses, zu verwenden, auf welche die Reaktionsmischung aufgegeben wird. Ebenso ist die Verwendung von Perlen, sogenannten beads, oder Latex-Partikeln möglich. Die feste Phase sollte mindestens soviele Bindungsstellen für die immobilisierbare Gruppe der Sonde haben, wie Nukleinsäurehybride D und damit Nukleinsäuren B vorhanden sind.

Die Herstellung einer bevorzugten festen Phase ist in der EP-A-0 344 578 beschrieben, auf welche vollinhaltlich Bezug genommen wird.

Nach einer Inkubationszeit, während der die Immobilisierungsreaktion stattfindet, wird die flüssige Phase aus dem Gefäß, dem porösen Material oder den pelletierten beads entfernt. Die Festphase kann anschließend mit einem geeigneten Puffer gewaschen werden, da die Bindung der Hybride D an der Festphase sehr effizient ist. Das erfindungsgemäße Verfahren erlaubt dabei die Verwendung besonders weniger Waschschritte, da die schwer abtrennbaren Sonden im Gegensatz zu im Stand der Technik verwendeten detektierbaren Sonden nicht unbedingt vollständig entfernt werden müssen, oder führt zu vergleichsweise geringen Hintergrundsignalen.

Die Menge der an die Festphase gebundenen modifizierten Nukleinsäuren kann auf im Prinzip bekannte Weise bestimmt werden, wobei sich die durchzuführenden Schritte nach der Art der nachweisbaren Gruppe richten. Bei direkt nachweisbaren Gruppen, beispielsweise Fluoreszenzlabeln, wird die Menge an Label fluorometrisch bestimmt. Ist die nachweisbare Gruppe ein Hapten, so wird die modifizierte Nukleinsäure bevorzugt mit einem markierten Antikörper gegen das Hapten umgesetzt, wie analog in der EP-A-0324474 beschrieben. Die Markierung kann beispielsweise eine Enzymmarkierung, wie β-Galaktosidase, alkalische Phosphatase oder Peroxidase sein. Im Fall der Enzymmarkierung wird die Menge an Nukleinsäure über die meist photometrische, chemoluminometrische oder fluorometrische Verfolgung einer Reaktion des Enzyms mit einem chromogenen, chemoluminogenen oder fluorogenen Substrat gemessen. Es ist jedoch auch elektrochemische Verfolgung der Reaktion möglich, wenn ein Redoxenzym als Markierung verwendet wird, oder die Verfolgung einer pH-Wertänderung mittels einer pH-Elektrode. Das Meßsignal ist ein Maß für die Menge an ursprünglich vorhandener target-Nukleinsäure und somit an nachzuweisenden Bakterien. In ersten Versuchen hat sich herausgestellt, daß sogar 1-5 Genom-äquivalente/Reaktion nachgewiesen werden können.

Der Nachweis der Nukleinsäure kann sowohl qualitativ als auch quantitativ geführt werden. Im Falle einer quantitativen Auswertung hat es sich als zweckmäßig herausgestellt, mindestens einen Vergleichsversuch mit einer Probe bekannten Nukleinsäuregehalts durchzuführen. Die Erstellung einer Eichkurve ist möglich und empfehlenswert.

In einer Ausführungsform des Verfahrens unter Verwendung der PCR werden der Probe als Primer P1 und P2 Oligonukleotide zugegeben. P1 ist hierbei komplementär zu einem Teil des Nukleinsäureeinzelstrangs A, welcher die target- und gleichzeitig die template-Nukleinsäure darstellt. P2 ist homolog zu einem davon entfernten Teil von A. Das Gemisch wird nun, wie in EP-A-0 201 184 beschrieben, behandelt, wobei jedoch neben den unmodifizierten Desoxymononukleotidtriphosphaten auch z.B. ein digoxigeninmarkiertes oder fluoresceinmarkiertes Desoxymononukleotidtriphosphat eingesetzt wird. Es werden bevorzugt 20-30 Amplifikationszyklen durchgeführt. Danach wird einzelsträngige biotinmarkierte Sonde C zugegeben. Die Mischung wird bei einer Temperatur zwischen 50 und 95°C, bevorzugt 80 und 95°C, besonders bevorzugt 85 bis 95°C ca. 1 bis 15 min inkubiert. Vorteil der thermischen Denaturierung auf dieser Stufe, gegebenenfalls nach mehreren Amplifikationsschritten ist, daß es wünschenswert ist, möglichst wenige Reagenzien zuzusetzen. Bei chemischer Denaturierung müßten im Extremfall sehr hohe Konzentrationen an solchen Reagenzien zugesetzt werden, z.B. um Pufferwirkungen, die auch durch die für den Aufschluß erforderlichen Reagenzien und deren Neutralisation entstanden sind, zu vermeiden. Dies kann auch bei der anschließenden Wandbindung der Hybride von Vorteil sein. Die Mischung wird, bevorzugt nach Abkühlung der Reaktionsmischung auf ca. 37°C, in ein Streptavidinbeschichtetes Gefäß umgefüllt und erneut inkubiert. Die Lösung wird entfernt und das Gefäß gewaschen. Es wird ein Konjugat aus Antikörper gegen Digoxigenin und einem Enzym zugegeben und erneut inkubiert. Nach Entfernen der Lösung und Waschen des Gefäßes wird mit einem chromogenen Substrat des Enzyms umgesetzt und die Farbbildung beobachtet. In den Verfahren des Standes der Technik wurden bisher immer nachweisbar markierte Sonden eingesetzt. Eine vollständige Abtrennung der nicht hybridisierenden Sonden war für die Genauigkeit des Meßergebnisses erforderlich, jedoch relativ aufwendig.

Das erfindungsgemäße Verfahren umgeht diesen Nachteil, indem anstelle der Hybridisierung von markierten Sonden und deren Bestimmung die Anwesenheit eingebauter markierter Mononukleotide gemessen und als Maß für die Anwesenheit bzw. die Menge an nachzuweisenden Nukleinsäuren genommen wird. Die Abtrennung nicht eingebauter markierter Mononukleotide ist nach dem vorliegenden Verfahren besonders einfach und vollständig zu erreichen, da sie weder an die Nukleinsäuren noch an die Oberfläche gebunden werden. Ein Überschuß an immobilisierbarer Sonde C hingegen stört die Bestimmung nicht, da die immobilisierbaren Gruppen von Sonde C nicht als Markierung verwendet werden und somit nicht zum Meßergebnis beitragen. Insbesondere ist die Bindekapazität der festen Phase besser kalkulierbar als beim Einbau von immobilisierbaren NTPs.

Das erfindungsgemäße Verfahren ist daher sehr sensitiv und selektiv, außerdem kann es in kurzer Zeit durchgeführt werden.

Das erfindungsgemäße Verfahren eignet sich zum Nachweis von Bakterienspezies, aber auch von taxonomischen Gruppen von Bakterien. Besonders gut verwendet werden kann das Verfahren bei der Bestimmung von pathogenen Mikroorganismen, wie z.B. von Salmonella spp., Listeria monocytogenes, Campylobacter spp., Vibrio parahaemolyticus, Vibrio cholerae, E.coli, Staphylococcus aureus, Clostridium perfringens, Clostridium botulinum, Bacillus spp., Yersinia enterocolytica. Der Nachweis ist bevorzugt möglich in Lebensmitteln, wie Milch, Milchprodukte wie Käse, Kefir, Joghurt, Fleisch, Fisch, Meeresfrüchte, Gemüse, Salat, Reis, Getreide, Eier, Geflügel, Gewürze, Kräuter und getrocknete Lebensmittel. Sowohl Ribonukleinsäuren als auch Desoxyribonukleinsäuren können nachgewiesen werden. Als target-Nukleinsäure sind Ribonukleinsäuren bevorzugt, wie rRNA; besonders bevorzugt 16 S oder 23 S-rRNA.

Im erfindungsgemäßen Verfahren ist es möglich, die Spezifität der spezifischen Starter und der Nukleinsäuresonde unterschiedlich zu wählen. Dadurch wird eine doppelte Spezifizierung möglich.

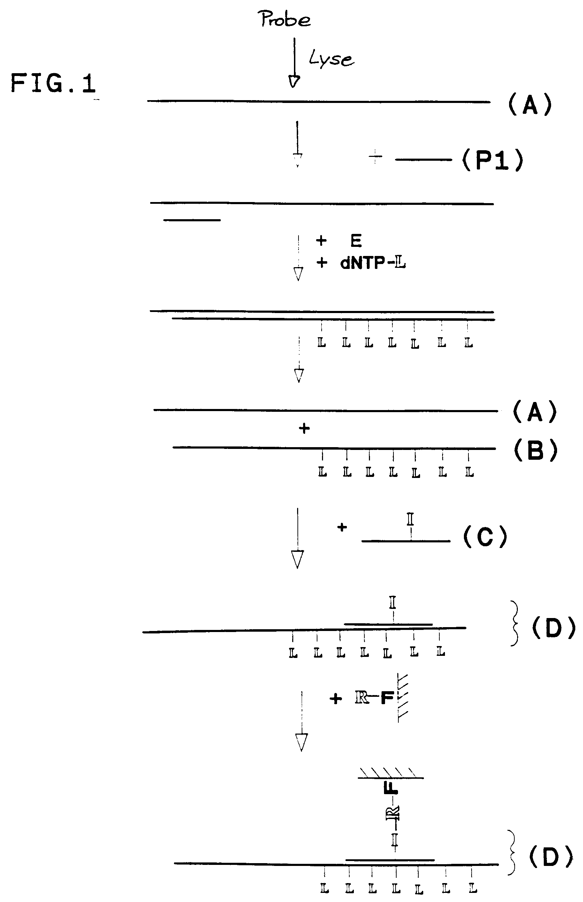

Figur 1 zeigt schematisch den Ablauf einer Ausführungsform unter Verwendung einer Primerelongation (Verwendung nur eines Primers P1).

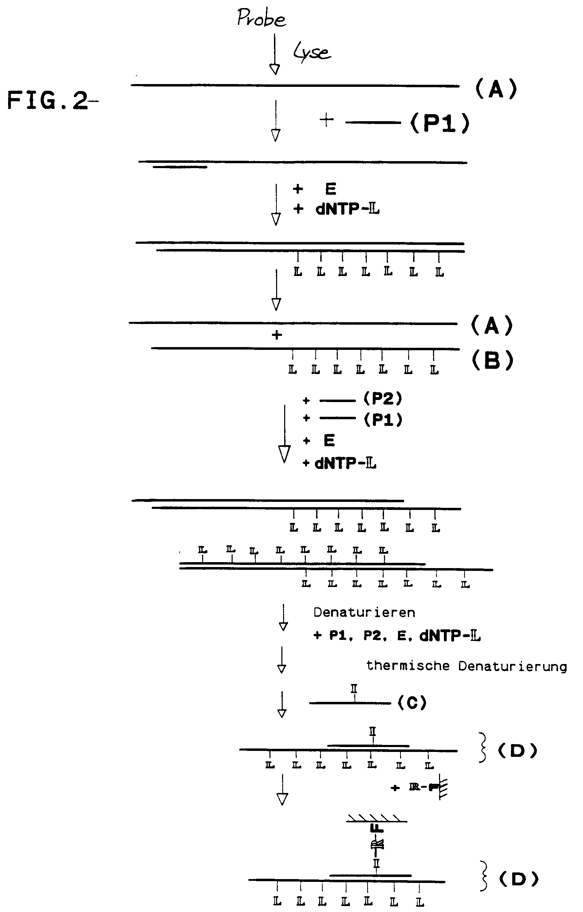

Figur 2 zeigt schematisch den Ablauf einer bevorzugten Ausführungsform unter Verwendung des PCR-Prinzips mit zwei Primern, wobei die zunächst gebildeten Nukleinsäuren B erneut als template-Nukleinsäure eingesetzt werden.

Figur 3 zeigt eine Eichkurve, die gemäß Beispiel 1 erhalten wurde.

Figur 4 zeigt die Nukleotidsequenz der in Beispiel 1 verwendeten Primer und der Sonde (DNA; linear; einzelsträngig; 20, 19 bzw. 20 bp).

Beispiel

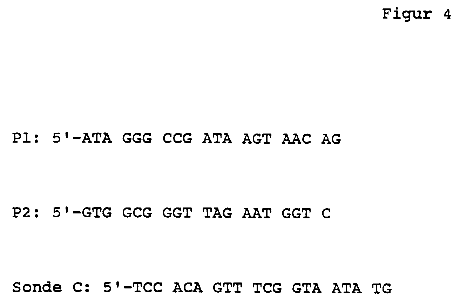

Die Bakterien werden miit 1% Triton X-100 unter 5 min Inkubation bei 95°C lysiert. 10 $\mu$l des Lysis-Ansatzes werden in einer polymerase chain reaction eingesetzt. Die verwendeten Primer binden an die Position 689-708 und 2223-2241 im 23 S rRNA-Gen von Listeria monocytogenes (Figur 4), d.h. eine Sequenz von 1552 Nukleotiden wird amplifiziert. In der hier eingesetzten Verdünnungsreihe wurden 5 fg bis 5 ng chromosomale DNA verwendet.

30 PCR-Zyklen (15" 94°C; 30" 60°C; 90" 72°C) werden in 100 $\mu$l mit Primern, je 200 mM; KCl, 50 mM; Tris HCl pH 8.5, 10 mM; MgCL$_2$. 1.5 mM; Gelatine 100 $\mu$g/ml; dATP, 200 $\mu$M; dGTP, 200 $\mu$M; dTTP, 150 $\mu$M; Digoxigenin-11-2'-desoxy-uridin-5'-dUTP (Dig-[11]-dUTP, Boehringer Mannheim), 50 $\mu$M; 2,5 U Thermus aquaticus (Taq) DNA-Polymerase durchgeführt. 20 $\mu$l des Amplifikationsansatzes und 40 ng Biotin-markierte Proben-DNA, Figur 4, welche an die Position 1191-1210 im oben genannten Gen bindet, werden in einem Gesamtvolumen von 40 $\mu$l 10 min bei 95°C denaturiert. Anschließend werden 160 $\mu$l Hybridisierungslösung (52.5 mM Natriumphosphat, pH 6.8; 6,25 x SSC [1 x SSC = NaCl, 0.15 M; Natriumcitrat, 0.015 M] und Formamid 62.5%) zugegeben und in eine Streptavidinbeschichtete Mikrotiterplatte umpipettiert. Die Hybridisierungs-/Wandbindung wird drei Stunden bei 37°C unter leichtem Schütteln durchgeführt. Nach Entfernen der Hybridisierungslösung und 3 x Waschen mit 0.9% NaCl werden 200 mU/ml 〈Digoxigenin〉-Meerrettich-Peroxidase-Konjugat zugegeben und 30 min bei 37°C in Tris HCl, pH 7.5, 10 mM; NaCl 0.9%, BSA 1%; Pluronic T68 0.5% inkubiert. Nach 3 x Waschen (Bedingungen siehe oben) wird mit 0.1% 2,2-Azino-di-[3-ethylbenzthiazol]-(ABTS) 30 min bei 37ºC inkubiert und die Extinktion bei 405 neu gemessen.

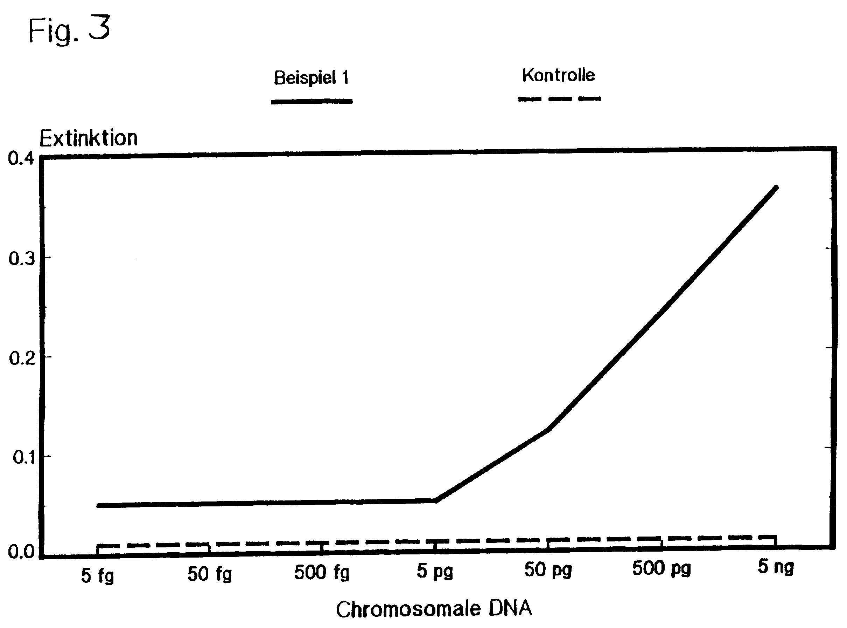

In Abbildung 3 sind die Extinktionen für diese Reaktion und für die Kontrollreaktion mit biomarkierter Probe gezeigt.

Anhand dieser Kurve können nun auch die in Proben mit unbekannter Bakterienkonzentration vorhandenen Nukleinsäurekonzentrationen und somit die Menge an Bakterien ermittelt werden.

Bezugszeichen:

A       template-Nukleinsäure
A1      Gegenstrang von A
P1      zu A komplementärer Primer
E       Enzym/Enzymkomplex
E1      DNA-Polymerase oder reverse Transskriptase
L       nachweisbare Gruppe (Markierung)
B       Produkt der Reaktion a)

C      Nukleinsäuresonde
I       immobilisierbare Gruppe
D      Nukleinsäurehybrid aus B und C
R      immobilisierende Gruppe
F      feste Phase
S1     zu A komplementäre Sequenz
S1'    andere zu A komplementäre Sequenz
S2     Promotor
S2'    Promotor

Die Erfindung wird durch das folgende Beispiel näher erläutert:

**Patentansprüche**

1. Verfahren zum spezifischen Nachweis eines Bakteriums in einer Probe, welches folgende Schritte umfaßt:

a) Aufschluß der Probe zur Freisetzung bakterieller Nukleinsäuren,

b) Reaktion der aufgeschlossenen Probe mit einem oder mehreren markierten Mononukleosidtriphosphaten und einem oder mehreren Enzymen, welche die Herstellung einer markierten Nukleinsäure B katalysieren, die dieses Nukleotid enthält,

c) Reaktion der Probe mit einer für das Bakterium spezifischen Nukleinsäuresonde C, welche hinreichend komplementär zu der Nukleinsäure B ist, und

d) Nachweis des aus markierter Nukleinsäure B und Nukleinsäuresonde C gebildeten Nukleinsäurehybrids D,

dadurch gekennzeichnet, daß,

e) die Nukleinsäuresonde mindestens eine immobilisierbare Gruppe enthält,

f) die Reaktionsmischung nach Schritt a) einer thermischen Denaturierung unterzogen wird,

g) das Nukleinsäurehybrid D mit einer festen Phase, welche die immobilisierbare Nukleinsäuresonde C spezifisch binden kann, in Kontakt gebracht wird,

h) die flüssige von der festen Phase getrennt wird und

i) die an die feste Phase gebundene nachweisbare Gruppe nachgewiesen wird,

wobei Verfahren ausgeschlossen sind, bei denen die bakteriellen Nukleinsäuresonden mit mindestens zwei Adaptoren pro Nukleinsäurestrang, von denen mindestens einer eine für ein Replikationssystem spezifische Nukleotidsequenz enthält, unter Bildung einer zu der nachzuweisenden Nukleinsäure im wesentlichen komplementären Nukleinsäure, die außerdem mindestens einen Adaptor enthält, umgesetzt werden.

2. Verfahren nach Anspruch 1, dadurch gekennzeichnet, daß Reaktion b) in Anwesenheit eines spezifischen Starters abläuft.

3. Verfahren gemäß Anspruch 1 oder 2, dadurch gekennzeichnet, daß das Enzym bzw. der Enzymkomplex die Polymerisation von Nukleosidtriphosphaten zu einer zu der Nukleinsäure A im wesentlichen komplementären Nukleinsäure B katalysiert.

4. Verfahren gemäß Anspruch 3, dadurch gekennzeichnet, daß die Nukleosidtriphosphate Ribonukleosidtriphosphate umfassen.

5. Verfahren gemäß Anspruch 2, dadurch gekennzeichnet, daß in Reaktion b) als spezifischer Starter ein Primer P1 benutzt wird, der zu einem Teil der Nukleinsäure A im wesentlichen komplementär ist und von dem Enzym unter Einbau des Mononukleosidtriphosphats zu einer zu der Nukleinsäure A im wesentlichen komplementären Nukleinsäure B verlängert wird.

6. Verfahren gemäß Anspruch 5, dadurch gekennzeichnet, daß außerdem ein Primer P2 benutzt wird, der zu einem Teil der Nukleinsäure B im wesentlichen komplementär ist.

7. Verfahren gemäß einem der Ansprüche 3 oder 5, dadurch gekennzeichnet, daß die Nukleosidtriphosphate Desoxyribonukleosidtriphosphate umfassen.

8. Verfahren nach einer der Ansprüche 1 bis 3, dadurch gekennzeichnet, daß die nachzuweisende Nukleinsäure einzelsträngig ist oder gemacht wird, der Probe dann mindestens ein Primer pro nachzuweisendem Einzelstrang zugesetzt wird, welcher eine zu einem Teil der nachzuweisenden Nukleinsäure im wesentlichen komplementäre Nukleotidsequenz und eine Transkriptionsinitiationssequenz enthält, der Primer um eine zu der nachzuweisenden Nukleinsäure komplementäre Nukleotidsequenz verlängert und die so gebildete Nukleinsäure als template-Nukleinsäure in Reaktion b) eingesetzt wird.

9. Verfahren gemäß einem der vorangehenden Ansprüche, dadurch gekennzeichnet, daß die in Schritt b) gebildete Nukleinsäure B als template-Nukleinsäure erneut in Reaktion b) eingesetzt wird.

10. Verfahren gemäß einem der vorangehenden Ansprüche, dadurch gekennzeichnet, daß Reaktion b) mehrmals hintereinander ausgeführt wird, wobei jeweils die Produkte der Reaktion wieder als Ausgangsstoffe der erneuten Reaktion dienen.

11. Verfahren gemäß Anspruch 9 oder 10, dadurch gekennzeichnet, daß in Reaktion b) ein Nukleinsäurehybrid aus Nukleinsäure A und Nukleinsäure B gebildet wird.

12. Verfahren gemäß einem der vorangegangenen Ansprüche, dadurch gekennzeichnet, daß die Denaturierung zwischen 50 und 95°C stattfindet.

13. Verfahren gemäß Anspruch 12, dadurch gekennzeichnet, daß die Nukleinsäuresonde C eine einzelsträngige Nukleinsäure C1 ist, deren Gegenstrang nicht in der Lösung vorhanden ist.

14. Verfahren nach Anspruch 6, dadurch gekennzeichnet, daß die durch Verlängerung der Primer P1 und P2 gebildeten Nukleinsäuren nach Strangtrennung erneut mit P1 und P2 umgesetzt werden, wobei die mit dem einen Primer neu gebildeten Stränge jeweils als template für die Verlängerung des anderen Primers dienen.

Probe

Lyse

FIG.1

(A)

(P1)

+ E
+ dNTP-L

L L L L L L L

(A)

+

(B)

L L L L L L L

+   II   (C)

II

(D)

L L L L L L L

+ R-F

F

R

II

(D)

L L L L L L L

FIG.2

# Fig. 3

Beispiel 1

Kontrolle

Extinktion

0.4

0.3

0.2

0.1

0.0

5 fg    50 fg    500 fg    5 pg    50 pg    500 pg    5 ng

Chromosomale DNA

EP 0 501 356 A1

Figur 4

P1: 5'-ATA GGG CCG ATA AGT AAC AG

P2: 5'-GTG GCG GGT TAG AAT GGT C

Sonde C: 5'-TCC ACA GTT TCG GTA ATA TG

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WO-A-9 011 374 (DU PONT DE NEMOURS) <br> * das ganze Dokument * <br> --- | 1-14 | C12Q1/68 |
| A | GB-A-2 202 328 (ORION-YHTYMA OY) <br> * das ganze Dokument * <br> --- | 1-14 | |
| P,A | EP-A-0 421 469 (CANON) <br> * das ganze Dokument * <br><br> ----- | 1-14 | |

| | RECHERCHIERTE SACHGEBIETE (Int. Cl.5 ) |
|---|---|
| | C12Q |

Der vorliegende Recherchenbericht wurde für alle Patentansprüche erstellt

| Recherchenort | Abschlußdatum der Recherche | Prüfer |
|---|---|---|
| DEN HAAG | 14 MAI 1992 | MOLINA GALAN E. |

EPO FORM 1503 03.82 (P0403)